# EUROPEAN PATENT APPLICATION

(11) **EP 4 679 055 A1**
(43) Date of publication of application: **14.01.2026**
(21) Application number: 24766337.0
(22) Date of filing: 29.02.2024
(51) Int. Cl.: G01N 1/28

(54) **URINE TREATMENT METHOD AND URINE DETECTION SYSTEM**

(30) Priority: 06.03.2023 CN 202310201948
(71) Applicant: Jiang, Hongtao, Shenzhen, Guangdong 518001 (CN); Jiang, Hongbo, Shenzhen, Guangdong 518001 (CN)
(72) Inventor: Jiang, Hongtao, Shenzhen, Guangdong 518001 (CN); Jiang, Hongbo, Shenzhen, Guangdong 518001 (CN)
(74) Representative: Metida
(86) International application number: PCT/CN2024/079443
(87) International publication number: WO 2024/183617

(57) **Abstract**

The present application provides a urine processing method and a urine testing system. The method comprises processing urine to obtain urinary sediment and supernatant, performing crystallization processing on the supernatant and making analysis to obtain supernatant-related parameters, and making comprehensive analysis through combination of the supernatant-related parameters with urinary sediment-related parameters and urine dry chemistry-related parameters. The system comprises a urine processing module configured to process urine to obtain urinary sediment and supernatant, and a supernatant analysis module configured to perform crystallization processing on the supernatant and make analysis to obtain supernatant-related parameters, and to make comprehensive analysis through combination of the supernatant-related parameters with the urinary sediment-related parameters and the urine dry chemistry-related parameters.

## Description

### TECHNICAL FIELD

The present application relates to the field of urine testing field, and more particularly to urine processing methods and urine testing systems.

### BACKGROUND

The academic community has established that the direct cause and formation process of urinary calculi mainly comprises the following three stages occurring in sequence: 1) concentration of dissolved calculus-forming components in urine becomes supersaturated; 2) precipitation of crystals of the corresponding components; and 3) continued growth and aggregation of crystals to form urinary calculi. Therefore, accurate evaluation and analysis of stages 1 and 2 are of significant importance for prevention and risk assessment of calculus formation.

Currently, the prevention and risk assessment method for urinary calculi that is widely recognized and recommended by the domestic and international medical communities is the 24-hour urine test. This method focuses on detecting the concentration of calculus-forming components, which involves collecting a patient's urine over a continuous 24-hour period and testing, the parameters tested include urine volume, pH value, and the concentration of related metabolites (potassium, calcium, sodium, magnesium, ammonium, chloride, phosphate, sulfate, nitrate, oxalate, citrate, and uric acid, etc.). Testing institutions can calculate degrees of supersaturation of oxalates, phosphates, uric acid, etc., based on specialized procedures. Despite the method's ability to accurately assess the concentration of calculus-related metabolites in the tested 24-hour-collected urine, it has several significant drawbacks as set forth herein below. 1) The test, which involves collecting urine multiple times over 24 hours, only reflects the overall metabolic indicators of the urine during this period and cannot accurately reflect the high calculus-forming risks associated with high concentrations of urine at certain periods, since calculus formation is the result of both the urolithogenesis due to high concentrations of the calculus-related metabolites in urine at different periods of a day and the accumulation of micro-calculus crystallization over a long period.Thus, the overall test of 24-hour-collected urine mainly reflects the general metabolic situation over 24 hours and cannot accurately reflect the urine conditions at different periods of a whole day, for example, the concentration of the calculus-related metabolites may be higher in morning urine. 2) The interpretation of test results is complex, and the assessment of urolithiasis etiology and urolithiasis risk is inaccurate, since the 24-hour urine test only provides the concentration of related substances and cannot directly reflect the risk level of specific calculus components. Even though specialized design methods and calculation processes can determine degrees of supersaturation of oxalates, phosphates and uric acid, etc., the assessment of lithogenic risk based on these calculated supersaturation indices is still affected by urine pH, ionic strength, other small molecules, and organic macromolecules, as urine is a complex mixture of solutions, and the interaction of the lithogenic substances is extremely complex . Accurate assessment is thus difficult, and there is no consistency in the calculation methods and model settings used by different testing institutions abroad. There are no relevant calculation methods domestically. 3) The testing process is complex and costly. The 24-hour urine test involves a wide variety of analytes, including multiple anions, cations, and related molecular substances, which may require different processing methods using various devices for separate detection, such as biochemical methods and chromatography. There is no single processing method that can test everything, and only a very small number of hospitals domestically have all the necessary testing equipment, and yet the calculation of degrees of supersaturation for oxalates, phosphates, and uric acid, etc., has not been carried out. 4) The test is inconvenient and the follow-up is difficult, as the 24-hour urine test requires carrying urine collection containers and collecting urine multiple times over a continuous 24-hour period, which is very inconvenient for daily life and work; additionally, since urine component is affected by diet, activities, climate, and other factors, a single test or a small number of tests cannot accurately assess the risk of calculus formation. This makes it very inconvenient for long-term monitoring of calculus-related urine indicators. Due to these disadvantages, the 24-hour urine test for calculus-related indicators is greatly limited. In developed countries, the usage rate among calculus patients is less than 10%, and it is even less commonly used domestically. This has led to difficulties in conducting and applying urolithiasis etiology screening and urolithiasis risk assessment.

The information included in the background section of the present application, including any references cited herein and their descriptions or discussions, is included solely for technical reference purposes and is not intended to be construed as limiting the scope of the present application.

### SUMMARY

In light of the aforementioned considerations, there is a need to develop a urine processing method and a urine testing system capable of detecting/assessing urolithiasis probability and urolithiasis risk. The inventors of such system and method innovatively propose to separate urinary sediment from supernatant and process them individually, and for the first time propose a crystallization-analysis method for urine supernatant. The method primarily focuses on a key step of urolithiasis etiology, namely the crystallization process, and comprehensively analyzes the first and second stages of calculus formation, which correspond to two main analytical parts: (i) Urinary Sediment Detection: If urine has already formed urinary sediment crystals, it indicates that the urine is in the second stage of calculus formation, with a high likelihood of calculus formation, and thus may be classified as high risk; (ii) Supernatant Detection: If there are no crystals in the urinary sediment, the supernatant is subjected to crystallization processing to determine whether urolithiasis-related crystals are formed, and to conduct qualitative and quantitative analysis for assessing the risk level of the first stage of calculus formation. The crystals formed in this first stage, compared to those present in the urinary sediment, pose a lower risk of calculus formation and can be classified as medium risk. If neither urinary sediment nor supernatant forms crystals, the likelihood of calculus formation is low. However, considering that urine of varying concentrations may be produced in different parts of each kidney, some local urine may have higher concentrations, and the potential crystallization risk cannot be completely ruled out, hence it can be considered low risk. The aforementioned method, based on the detection and analysis of urinary crystals and crystallization process, provides clinically meaningful parameters for the urolithiasis etiological screening and the urolithiasis risk assessment. Compared with the 24-hour urine test, the technical advantages of the present application include: (1) more direct and accurate analysis of of urolithiasis etiology and assessment of urolithiasis risk; (2) simpler and clearer interpretation and explanation of test results; (3) detection and analysis method and system can be integrated, resulting in a more streamlined and efficient testing process; (4) lower overall equipment cost and single-test cost, making multiple follow-ups for urolithiasis and long-term monitoring of urolithiasis recurrence more convenient and economical.

In accordance with one aspect of the present application, a urine processing method is provided, which comprises the following steps: S1: processing urine to obtain urinary sediment and supernatant; and S2: performing crystallization processing on the supernatant and making analysis to obtain supernatant-related parameters.

In one embodiment, in the step S2, performing crystallization processing on the supernatant until supernatant crystals are formed in the supernatant, thereafter making qualitative and/or quantitative analysis of the supernatant crystals to obtain supernatant-related parameters, the supernatant-related parameters including types and/or quantities of the supernatant crystals.

In one embodiment, performing crystallization processing on the supernatant at a temperature of -10 - 55°C.

In one embodiment, performing evaporation-induced crystallization processing on the supernatant at a temperature of 4 - 55°C.

In one embodiment, performing either cold-induced crystallization processing on the supernatant , or alternatively performing both evaporation-induced crystallization and cold-induced crystallization processing on the supernatant, at a temperature of -10 - 4°C.

In one embodiment, the method further comprises making dry chemistry analysis of the urine to obtain dry chemistry-related parameters.

In one embodiment, identifying whether sediment crystals exist in the urinary sediment, and making analysis of the sediment crystals, if existed, to obtain urinary sediment-related parameters.

In one embodiment, making qualitative and/or quantitative analysis of the sediment crystals, to obtain urinary sediment-related parameters, the urinary sediment-related parameters including types and/or quantities of the sediment crystals.

In one embodiment, the method further comprises, assessing urinary calculus components and urolithiasis risk, based on at least one of the supernatant-related parameters and the urinary sediment-related parameters.

In one embodiment, the method further comprises, assessing urinary calculus components and urolithiasis risk, based on at least one of the supernatant-related parameters and the urinary sediment-related parameters, as well as the dry chemistry-related parameters.

In one embodiment, the method is the one for processing single urine sample or combined multiple urine samples, wherein in the step S1, processing only single urine sample or alternatively combined multiple urine samples to obtain the urinary sediment and the supernatant.

In accordance with an aspect of the present application, a urine testing system is provided, which comprises: a urine processing module configured to process urine to obtain urinary sediment and supernatant; and a supernatant analysis module configured to perform crystallization processing on the supernatant and make analysis to obtain supernatant-related parameters.

In one embodiment, in case the supernatant crystals are formed in the supernatant upon crystallization processing, the supernatant analysis module configured to make qualitative and/or quantitative analysis of the supernatant crystals to obtain supernatant-related parameters, the supernatant-related parameters including types and/or quantities of the supernatant crystals.

In one embodiment, the urine processing module is configured to perform crystallization processing on the supernatant at a temperature of -10 - 55°C.

In one embodiment, the urine processing module is configured to perform evaporation-induced crystallization processing on the supernatant at a temperature of 4-55°C.

In one embodiment, the urine processing module is configured to perform either cold-induced crystallization processing on the supernatant or alternatively perform both evaporation-induced crystallization and cold-induced crystallization processing on the supernatant, at a temperature of -10-4°C.

In one embodiment, the system further comprises a dry chemistry-related parameter acquisition module configured to make dry chemistry analysis of the urine to obtain dry chemistry-related parameters.

In one embodiment, the system further comprises a urinary sediment analysis module configured to identify whether sediment crystals exist in the urinary sediment, and to make analysis of the sediment crystals to obtain urinary sediment-related parameters.

In one embodiment, the system further comprises a data processing module configured to assess the urinary calculus components and the urolithiasis risk, based on at least one of the supernatant-related parameters and the urinary sediment-related parameters.

In one embodiment, the system further comprises a data processing module configured to comprehensively assess the urinary calculus components and the urolithiasis risk, based on at least one of the supernatant-related parameters and the urinary sediment-related parameters, as well as the dry chemistry-related parameters.

In one embodiment, making qualitative and/or quantitative analysis of the sediment crystals, to obtain urinary sediment-related parameters, the urinary sediment-related parameters including types and/or quantities of the sediment crystals.

In one embodiment, the data processing module is configured to: assess the urolithiasis risk as high risk when the sediment crystals are identified in the urinary sediment; assess the urolithiasis risk as medium risk when the sediment crystals are not identified in the urinary sediment, and the supernatant crystals formed from the supernatant contain the urolithiasis-related crystals; and assess the urolithiasis risk as low risk when the sediment crystals are not identified in the urinary sediment, and the supernatant crystals formed from the supernatant contain none of the urolithiasis-related crystals.

In one embodiment, the urine processing module is configured to process single urine sample or combined multiple urine samples to obtain the urinary sediment and the supernatant.

In one embodiment, the supernatant analysis module is configured to identify, through Raman spectroscopy, infrared (IR) spectroscopy, or image recognition, whether the supernatant crystals contain at least one of the following urolithiasis-related crystals: CaOx (calcium oxalate), UA (uric acid), CaP ( calcium phosphate or brushite), ST (ammonium magnesium phosphate or triple phosphate), and CYS (cystine).

In one embodiment, the supernatant analysis module is configured to identify through image recognition technology, whether the supernatant crystals contain at least one of the following urolithiasis-related crystals: CaOx (calcium oxalate), UA (uric acid), CaP ( calcium phosphate or brushite), ST (ammonium magnesium phosphate or triple phosphate), and CYS (cystine), the image recognition comprises: identifying a crystal as calcium oxalate when the image of said crystal is of at least one of shapes comprising octahedral shape, equilateral rhombic shape, elliptical shape, dumbbell shape, biconvex columnar shape, and drum shape; identifying a crystal as uric acid when the image of said crystal is of at least one of shapes comprising irregular rhombic shape, square shape, blocky shape, multilayered lamellar shape, and petal shape; identifying a crystal as calcium phosphate or brushite when the image of said crystal is of at least one of shapes comprising rod shape, bar shape, fagot shape, feather shape, and chrysanthemum shape; identifying a crystal as ammonium magnesium phosphate or triple phosphate when the image of said crystal is of at least one of shapes comprising box-lid shape, roof shape, envelope shape, and coffin-lid shape; and identifying a crystal as cystine when the image of said crystal is of at least one of shapes comprising regular hexagonal shape and multilayered hexagonal shape.

The present application, through special processing and technical analysis of urine, enables ultimate output of the physical and chemical parameters of the urine and the qualitative and quantitative results of the urolithiasis-related crystals, revealing the urolithiasis risk in the urine sample. This provides a basis for the urolithiasis etiology analysis and the prevention of urolithiasis. Compared to traditional methods, the present application provides a method and a system for urolithiasis etiology analysis and assessment of urolithiasis risk in a more direct and accurate way; simpler and clearer interpretation and explanation of test results; integrated detection and analysis method and system, resulting in a more streamlined and efficient testing process; and lower overall system and equipment costs, and lower single-test costs, making multiple follow-ups for urolithiasis and long-term monitoring of urolithiasis recurrence more convenient and economical.

### BRIEF DESCRIPTION OF THE DRAWINGS

The above-mentioned features and advantages of these embodiments, as well as other features and advantages and the ways in which they are implemented, will become more apparent through reference to the following description in conjunction with the drawings, and the embodiments of the present application can be better understood.
FIG. 1 schematically illustrates a flowchart of a urine processing method according to an embodiment of the present application.
FIG. 2 schematically illustrates an embodiment of a urine testing system for implementing the urine processing method shown in FIG. 1.
FIGs. 3A-3F schematically illustrate common urolithiasis-related crystalline morphologies of urinary sediment obtained according to the method shown in FIG.1: CaOx, UA, CaP, ST, and CYS.
FIGs. 4A-4H schematically illustrate common urolithiasis-related crystalline morphologies of supernatant obtained according to the method shown in FIG. 1: CaOx, UA, CaP, ST, and CYS.

### DETAILED DESCRIPTION

In the following description of the drawings and specific modes of implementation, the details of one or more embodiments of the present application will be set forth. From these descriptions, drawings, and claims, the other features, objectives and advantages of the present application can be clearly understood.

It should be understood that the embodiments illustrated and described are not limited to the details of the construction and arrangement of the components as described below. The illustrated embodiments can be other embodiments that can be implemented or carried out in various ways. The examples are provided by way of explanation of the disclosed embodiments and not by way of limitation. Indeed, it will be apparent to those skilled in the art that various modifications and variations can be made to the embodiments of the present application without departing from the scope or spirit of the present disclosure. For example, features illustrated or described as part of one embodiment may be used with another embodiment to form yet another embodiment. Therefore, the present application covers such modifications and variations within the scope of the appended claims and their equivalents.

Similarly, it should be understood that the phrases and terms used in the text are for descriptive purposes and should not be considered restrictive. The wordings "comprising", " including", "having" and variations thereof used herein are for the purpose of description in an open manner and intended to contain items listed thereafter and equivalents thereof, as well as additional items if appropriate. The term "urinary sediment" refers to the solid phase formed upon urine separation. The term "supernatant" refers to the liquid phase formed upon urine separation.

The term "crystallization processing" refers to the process for inducing crystal formation. The terms "supernatant crystal" and "sediment crystal" are used to distinguish the source of the crystals, thus supernatant crystal and sediment crystal may be of the same type or different types of crystals.

More detailed description of the present application will now be provided below with reference to specific embodiments of the present application.

### Embodiment 1

Multiple patients with history of urolithiasis and calculus analysis reports were recruited. The morning urine of each patient was taken for urine dry chemistry analysis, urinary sediment crystallization analysis, and supernatant crystallization analysis, and compared with the patient's calculus analysis results to see if they contained the same type of crystals.

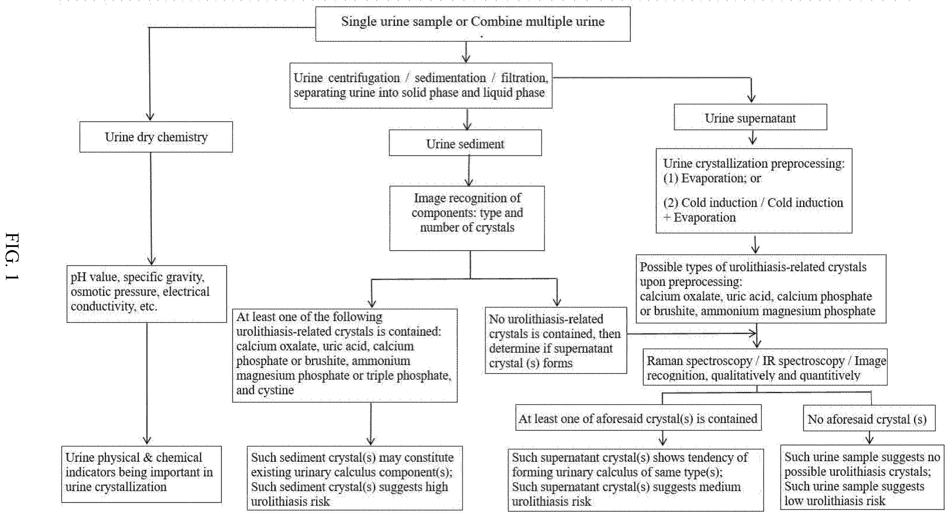

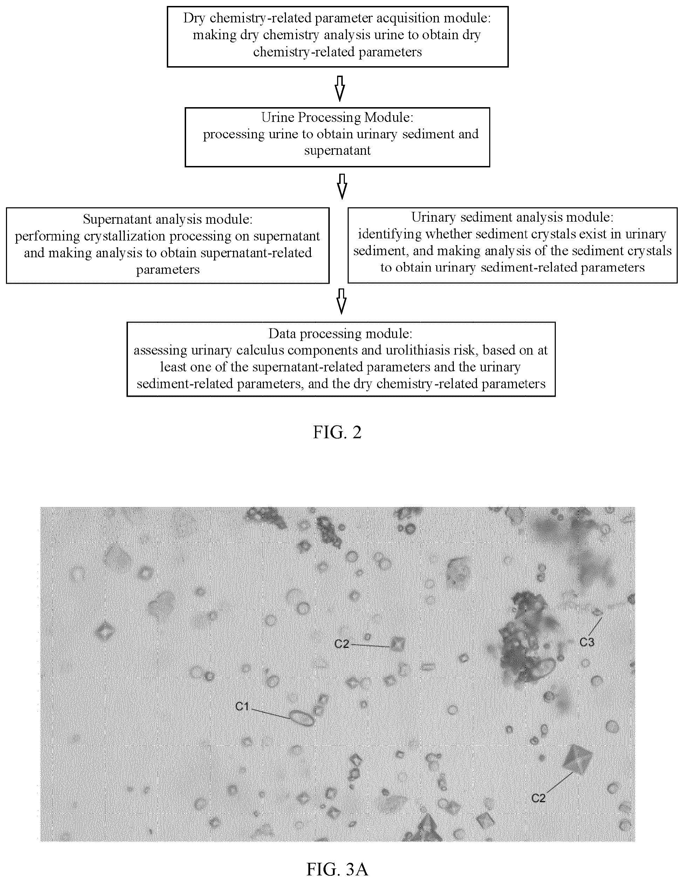

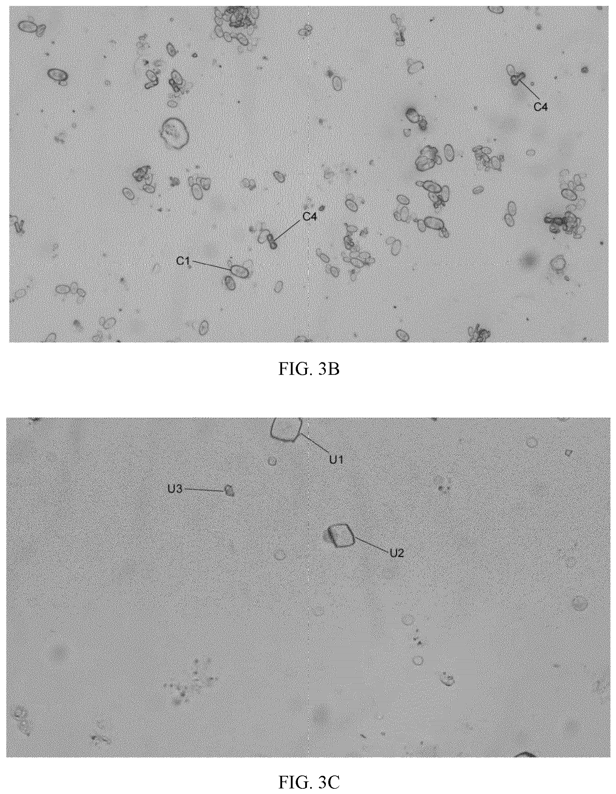

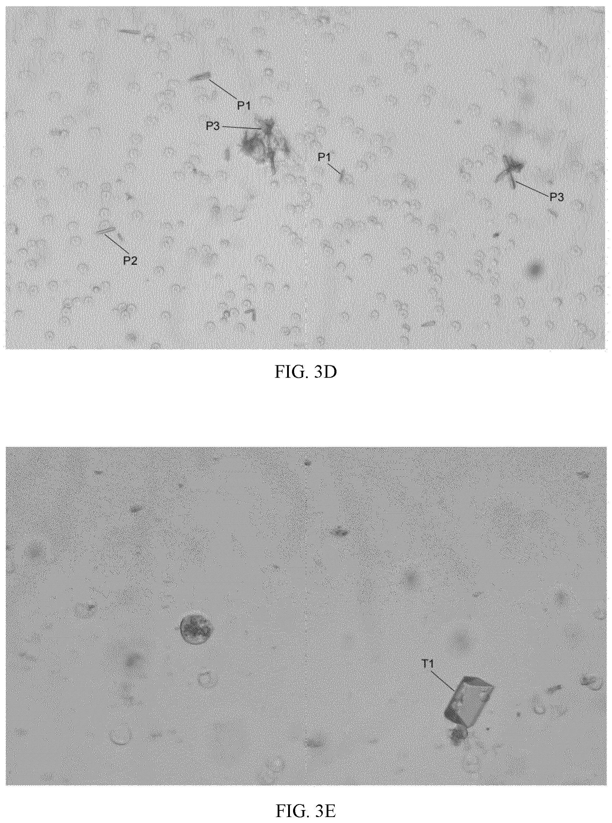

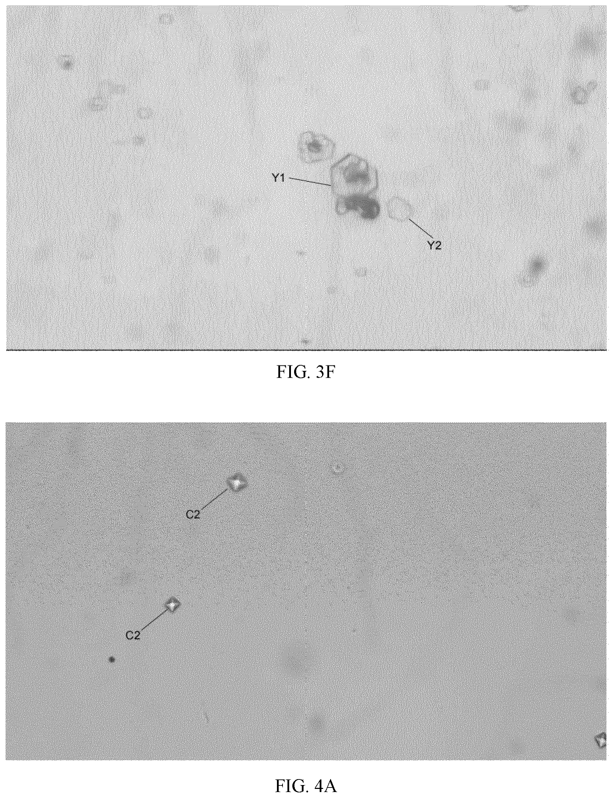

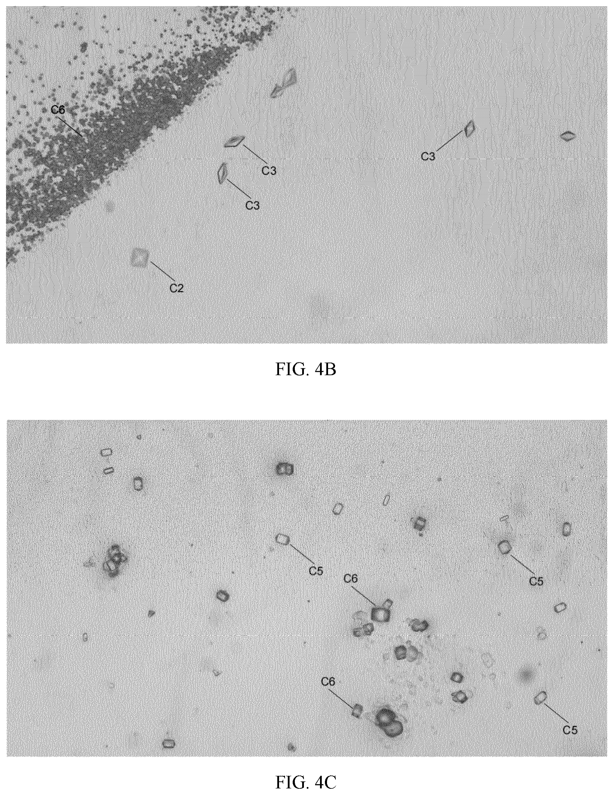

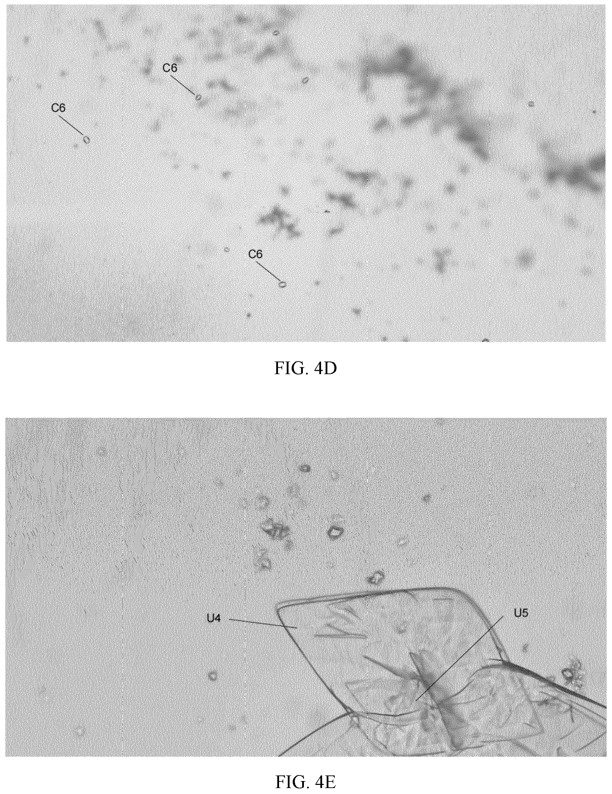

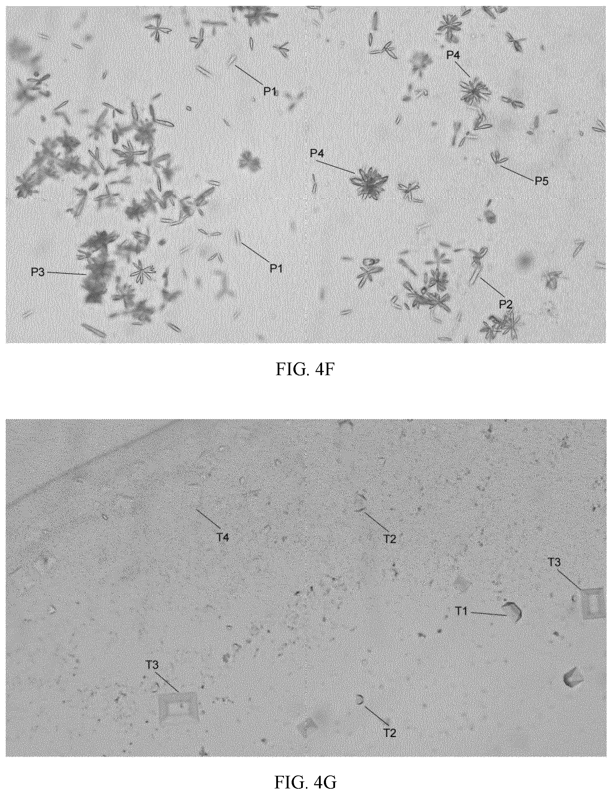

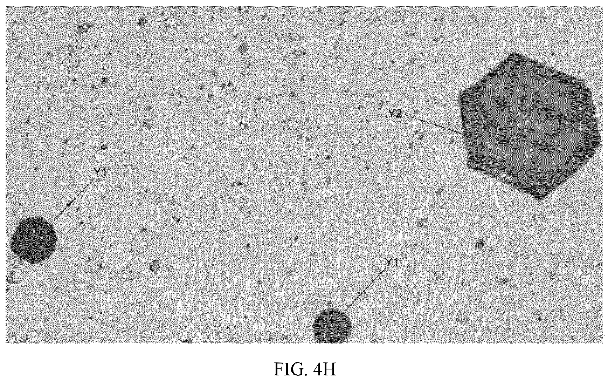

The test steps are shown in FIG. 1 and are specifically as follows:
(1) Urine was subjected to dry chemistry analysis to obtain indicators such as pH value, specific gravity, osmotic pressure, and electrical conductivity of the urine. Urine dry chemical indicators play an important role in urolithogenesis and urolithiasis recurrence risk control. For example, an acidic pH value tends to induce formation of calcium oxalate, uric acid, and cystine calculi, while an alkaline pH value tends to induce formation of calcium phosphate and magnesium ammonium phosphate calculi. A high specific gravity and a high osmotic pressure indicate a higher solute concentration in the urine, and a high electrical conductivity indicates a higher concentration of electrolytes in the urine, all of which are risk factors influencing the urolithogenesis and the urolithiasis recurrence.
(2) 5 ml of urine was drawn and centrifuged using a centrifuge device (such as, but not limited to, the low-speed centrifuge LC-32 from Anhui Zhongke Zhongjia Scientific Instrument Co., Ltd.), or separated by sedimentation, filtration, etc., into a solid phase (urinary sediment) and a liquid phase (supernatant).
(3) Conducting qualitative and/or quantitative analysis of sediment crystals in the urinary sediment by image recognition (such as, but not limited to, through an Olympus optical microscope and a digital camera), or analysis by Raman spectroscopy (such as, but not limited to, through a micro-Raman spectrometer). Qualitative analysis determines the presence and the type of sediment crystals, with the presence of crystals indicating a high risk, and analyzes the type of sediment crystals (common crystal types in urinary sediment are shown in FIGs. 3A-3F, where FIG. 3A shows CaOx crystals in the form of octahedral C2, equilateral rhombic C3, and elliptical C1; FIG. 3B shows CaOx crystals in the form of elliptical C1 and dumbbell shaped C4; FIG. 3C shows UA crystals in the form of irregular rhombic U3, square U1, and blocky U2; FIG. 3D shows CaP crystals in the form of rod shaped P1, bar shaped P2, and fagot shaped P3; FIG. 3E shows ST crystals in the form of roof shaped T1; FIG. 3F shows CYS crystals in the form of regular hexagonal Y2 and multilayered hexagonal Y1). Quantitative analysis involves counting and measuring the size of crystals that are positive (+) in qualitative analysis as well as other statistical treatment, and obtaining urine sediment-related parameters through qualitative and quantitative analysis.
(4) The supernatant was processed and analyzed, by drawing the supernatant of the urine and then evaporating it at temperatures of 10-37°C, such as at 10°C, 25°C, and 37°C, to induce formation of supernatant crystals through water loss. The formed supernatant crystals were subjected to qualitative analysis by Raman spectroscopy, infrared spectroscopy, or image recognition, to identify the type, chemical component, and crystal structure of the supernatant crystals (common urolithiasis-related crystal types in supernatant are shown in FIGs. 4A-4H, wherein FIG. 4A shows CaOx crystals in the form of octahedral C2; FIG. 4B shows CaOx crystals in the form of equilateral rhombic C3 and octahedral C2; FIG. 4C shows CaOx crystals in the form of biconvex columnar C5 and drum shaped C6; FIG. 4D shows CaOx crystals in the form of drum shaped C6; FIG. 4E shows UA in the form of multilayered lamellar U4 and petal shaped U5; FIG. 4F shows CaP crystals in the form of rod shaped P1, bar shaped P2, fagot shaped P3, feather shaped P5, and chrysanthemum shaped P4; FIG. 4G shows ST crystals in the form of box-lid shaped T2, roof shaped T1, envelope shaped T4, and coffin-lid shaped T3; FIG. 4H shows CYS crystals in the form of regular hexagonal Y2 and multilayered hexagonal Y1), and to obtain supernatant-related parameters through quantitative analysis of crystal counting, crystal size and area proportion via image recognition.

According to the current research and theoretical consensus in the medical community on the urolithogenesis (such as the references of "International Urolithiasis Alliance: Urolithiasis Metabolism and Management Guidelines 2022"), the urolithogenesis, i.e., the formation of urinary calculi, is closely related to the sediment crystals and the saturation degree of calculus-related components in urine, that is, (1) the sediment crystals in urine sediment are crystals that already exist in urine, indicating that the components of these crystals are already oversaturated in urine (at least locally oversaturated while secreted by local parts of kidney to form urine), and are an important cause of urinary calculus formation, (2) the oversaturation of calculus-related components in supernatant is prone to induce precipitatation and formation of crystals, which is thus closely related to urolithogenesis.

In this Embodiment, urine samples with sediment crystals in the urinary sediment, and urine samples without sediment crystals in the urinary sediment but with urolithiasis-related crystals in the supernatant, were selected respectively, and these crystals were compared and analyzed with the patient's urinary calculus components of corresponding urine samples, respectively. The analysis results are listed as follows in Table 1.

**Table 1. Supernatant Crystallization of Urine samples with Sediment Crystals and Calculus Analysis Results**

| | | Respective Numbers in Urine samples and in Patient's Calculus Components | | | | |
|---|---|---|---|---|---|---|
| | | CaOx | UA | CaP | ST | CYS |
| Sediment Crystals (+) | | 52 | 8 | 3 | 2 | 1 |
| Supernatant Crystals | (+) | 52 | 8 | 3 | 2 | 1 |
| | (-) | 0 | 0 | 0 | 0 | 0 |
| IR Analysis of Patient's Calculus Components | (+) | 51 | 8 | 3 | 2 | 1 |
| | (-) | 1 | 0 | 0 | 0 | 0 |

| | | | | | | |
|---|---|---|---|---|---|---|
| (+) indicates the presence of crystals, (-) indicates the absence of crystals. | | | | | | |

Table 1 shows that in 52 urine samples with CaOx crystals in the sediment, supernatant crystals CaOx were detected in all 52 samples, and IR analysis (infrared analysis) of patient's calculus components have detected CaOx in 51 patient's urinary calculus components, with only 1 exception (calculus component analysis did not detect out CaOx in only 1 patient's sample of urinary calculus components). In 8 urine samples with UA crystals in the sediment, supernatant crystals UA are detected in all the 8 samples, and IR analysis of calculus components detected UA in all 8 patient's urinary calculus components. In 3 urine samples with CaP crystals in the sediment, supernatant crystals CaP are detected in all these 3 samples, and IR analysis of calculus components detected CaP in all 3 patient's urinary calculus components. In 2 urine samples with ST crystals in the sediment, supernatant crystals ST are detected in these 2 urine samples, and IR analysis of calculus components detected ST in all 2 patient's urinary calculus components. In 1 urine sample with CYS crystals in the sediment, supernatant crystals CYS are detected in that 1 sample, and IR analysis of calculus components detected CYS in the patient's urinary calculus components of that 1 sample.

In summary, in this embodiment, when any of the 5 types of urolithiasis-related crystals are present (+) in the sediment, the supernatant will show the same type of crystals 100% of the time, and the probability that the patient's calculus components contain the same component (s) is extremely high, almost 100% (out of a total of 66 patients, only 1 is an exception).

**Table 2. Urine samples without Sediment Crystals but with Supernatant Crystals and Calculus Analysis Results**

| | | Respective Numbers in Urine samples and in Patient's Calculus Components | | | | |
|---|---|---|---|---|---|---|
| | | CaOx | UA | CaP | ST | CYS |
| Sediment Crystals (-) | | 126 | 3 | 26 | 5 | 2 |
| Supernatant Crystals | (+) | 126 | 3 | 26 | 5 | 2 |
| | (- ) | 0 | 0 | 0 | 0 | 0 |
| IR Analysis of Patient's Calculus Components | (+) | 118 | 2 | 21 | 4 | 2 |
| | (- ) | 8 | 1 | 5 | 1 | 0 |

Table 2 shows that in 126 urine samples without CaOx crystals in the sediment but with CaOx detected in supernatant crystals, and IR analysis of calculus components detected CaOx in 118 patient's urinary calculus components, with only 8 exceptions ( No CaOx being detected in 8 patient's urinary calculus components). In 3 urine samples without UA crystals in the urine sediment but with UA crystals detected in supernatant crystals, and IR analysis of calculus components detected UA in 2 patient's urinary calculus components, with 1 exception without UA detected. In 26 urine samples without CaP crystals in the sediment but with CaP detected in supernatant crystals, and IR analysis of calculus components detected CaP in 21 patient's urinary calculus components, with 5 exceptions. In 5 urine samples without ST crystals in the sediment but with ST detected in supernatant crystals, and IR analysis of calculus components detected ST in 4 patient's urinary calculus components, with 1 exception. In 2 urine samples without CYS crystals in the sediment but with CYS detected in supernatant crystals, and IR analysis of calculus components detected CYS in all 2 patient's urinary calculus components.

In summary, when no urolithiasis-related crystals (-) are present in the sediment , but a certain type of urolithiasis-related crystal appears (+) in the supernatant, there is a higher probability of consistency with the patient's calculus component analysis. That is, if any one of CaOx, UA, CaP, ST, and CYS crystals is present (+) in the supernatant crystals, the likelihood of the same component contained in urinary calculus of the corresponding patient is 93.7%, 66.7%, 80.8%, 80.0%, 100%, respectively.

The comprehensive results of this embodiment indicate that, (1) when a certain type of urolithiasis-related crystal is already present (+) in the urinary sediment, it indicates stage 2 of calculus formation, that is, this type of solid crystal in the urine may further form calculi, and the urolithiasis risk for this type of component is classified as high risk; (2) if no urolithiasis-related crystals are present in the urinary sediment, but a certain type of urolithiasis-related crystal appears (+) in the supernatant upon processing, it indicates stage 1 of calculus formation, the urine has the potential to form this type of crystal, and the urolithiasis risk for this type of component is classified as medium risk; (3) when neither urinary sediment nor supernatant contains urolithiasis-related crystals, but considering that the concentration of urine produced by different parts of each kidney is not completely consistent, there is still a possibility of higher concentration urine in some kidney areas, and the possibility of crystal formation cannot be completely ruled out, therefore, the urolithiasis risk is classified as low risk. Urine dry chemistry, pH value, specific gravity, osmotic pressure, electrical conductivity, etc., are auxiliary indicators and factors that promote or inhibit the formation of a specific type of crystal and its subsequent induction of urolithogenesis.

### Embodiment 2

Embodiment 2 is essentially the same as Embodiment 1, with the main difference being that the supernatant crystallization processing in Embodiment 2 is carried out at a temperature of 4-10°C, such as at 4°C, with the results as shown in the tables below. The urine dry chemistry and urinary sediment detection are substantially the same as in Embodiment 1.

In this Embodiment 2, urine samples with sediment crystals in the urinary sediment, and urine samples without sediment crystals in the urinary sediment but with urolithiasis-related crystals in the supernatant, were selected respectively, and these crystals were compared and analyzed with the patient's urinary calculus components of corresponding urine samples, respectively. The analysis results are listed as follows.

**Table 3. Supernatant Crystallization of Urine samples with Sediment Crystals and Calculus Analysis Results**

| | | Respective Numbers in Urine samples and in Patient's Calculus Components | | | | |
|---|---|---|---|---|---|---|
| | | CaOx | UA | CaP | | |
| Sediment Crystals (+) | | 25 | 3 | 2 | | |
| Supernatant Crystals | (+) | 25 | 3 | 2 | | |
| | (- ) | 0 | 0 | 0 | | |
| IR Analysis of Patient's Calculus Components | (+) | 25 | 3 | 1 | | |
| | (- ) | 0 | 0 | 1 | | |

Table 3 shows that in 25 urine samples with CaOx crystals in the sediment, supernatant crystals CaOx were detected in all 25 samples, and IR analysis of calculus components of patients have detected CaOx in all 25 patient's urinary calculus components, with no exceptions (0 urinary calculus component did not contain CaOx components in the analysis). In 3 urine samples with UA crystals in the sediment, supernatant crystals UA were detected in all 3 samples, and IR analysis of calculus components detected UA in all 3 patient's urinary calculus components. In 2 urine samples with CaP crystals in the sediment, supernatant crystals CaP were detected in these 2 urine samples, and IR analysis of calculus components detected CaP in 1 patient's urinary calculus components.

In summary, when any of the 3 types of urolithiasis-related crystals are present (+) in the sediment, the supernatant will show the same type of crystals 100% of the time, and the probability that the patient's calculus components contain the same crystal component is extremely high (out of a total of 30 patients, only 1 is an exception).

**Table 4. Urine samples without Sediment Crystals but with Supernatant Crystals and Calculus Analysis Results**

| | | Respective Numbers in Urine samples and in Patient's Calculus Components | | | | |
|---|---|---|---|---|---|---|
| | | CaOx | UA | CaP | | |
| Sediment Crystals (-) | | 20 | 4 | 2 | | |
| Supernatant Crystals | (+) | 20 | 4 | 2 | | |
| | (- ) | 0 | 0 | 0 | | |
| IR Analysis of Patient's Calculus Components | (+) | 18 | 3 | 1 | | |
| | (- ) | 2 | 1 | 1 | | |

Table 4 shows that in 20 urine samples without CaOx crystals in the sediment but with CaOx detected in supernatant crystals, and IR analysis of calculus components detected CaOx in 18 calculus components of patients, with only 2 exceptions (CaOx component were not detected in 2 calculus components of patients). In 4 urine samples without UA crystals in the sediment but with UA detected in supernatant crystals, and IR analysis of calculus components detected UA in 3 calculus components of patients. In 2 urine samples without CaP crystals in the sediment but with CaP detected in supernatant crystals, and IR analysis of calculus components detected CaP in 1 calculus component of patient, with 1 exception.

In summary, when urolithiasis-related crystals are not present (-) in the sediment, but a certain type of urolithiasis-related crystal appears (+) in the supernatant, there is a higher probability of consistency with the corresponding patient's calculus component analysis. That is, if any one of CaOx, UA, CaP crystals is present (+) in the supernatant crystals, the likelihood of the same component contained in urinary calculus of the corresponding patient is 90.0%, 75.0%, 50.0%, respectively.

The comprehensive results of this Embodiment indicate that, (1) when a certain type of urolithiasis-related crystal is already present (+) in the urinary sediment, it indicates stage 2 of calculus formation, that is, this type of solid crystal in the urine may further form calculi, and the urolithiasis risk for this type of component is classified as high risk; (2) if no urolithiasis-related crystals are present in the urinary sediment, but a certain type of urolithiasis-related crystal appears (+) in the supernatant after processing, it indicates stage 1 of calculus formation, the urine has the potential to form this type of crystal, and the urolithiasis risk for this component is classified as medium risk; (3) when neither urinary sediment nor supernatant contains urolithiasis-related crystals, but considering that the concentration of urine produced by different parts of each kidney is not completely consistent, there is still a possibility of higher concentration urine in some kidney areas, and the possibility of crystal formation cannot be completely ruled out, therefore, the urolithiasis risk is classified as low risk. Urine dry chemistry, pH value, specific gravity, osmotic pressure, electrical conductivity, etc., are auxiliary indicators and factors that promote or inhibit the formation of a specific type of crystal and its subsequent induction of urolithogenesis.

### Embodiment 3

Embodiment 3 is essentially the same as Embodiment 1, with the main difference being that the supernatant crystallization processing in Embodiment 3 can be carried out at temperatures of -10 - 4°C, such as at - 10°C and 4°C, with the results as shown in the tables below. The urine dry chemistry and urinary sediment detection are substantially the same as in the previous Embodiments.

In this Embodiment, urine samples with sediment crystals in the urinary sediment, and urine samples without sediment crystals in the urinary sediment but with urolithiasis-related crystals in the supernatant, were selected respectively, and these crystals were compared and analyzed with the patient's urinary calculus components of corresponding urine samples, respectively. The analysis results are listed as follows.

**Table 5. Supernatant Crystallization of Urine samples with Sediment Crystals and Calculus Analysis Results**

| | | Respective Numbers in Urine samples and in Patient's Calculus Components | | | | |
|---|---|---|---|---|---|---|
| | | CaOx | UA | CaP | ST | CYS |
| Sediment Crystals (+) | | 17 | 3 | 2 | 1 | 1 |
| Supernatant Crystals | (+) | 17 | 3 | 2 | 1 | 1 |
| | (- ) | 0 | 0 | 0 | 0 | 0 |
| IR Analysis of Patient's Calculus Components | (+) | 15 | 3 | 2 | 1 | 1 |
| | (- ) | 2 | 0 | 0 | 0 | 0 |

Table 5 shows that in 17 urine samples with CaOx crystals in the sediment, supernatant crystals CaOx were detected in all 17 samples, and IR analysis of calculus components detected CaOx in 15 patient's urinary calculus components, with 2 exceptions (in 2 patient's urinary calculus components, CaOx components were not detected). In 3 urine samples with UA crystals in the sediment, supernatant crystals UA were detected in all 3 urine samples, and IR analysis of calculus components detected UA in all 3 patient's urinary calculus components. In 2 urine samples with CaP crystals in the sediment, supernatant crystals CaP were detected in these 2 urine samples, and IR analysis of calculus components detected CaP were detected in all the 2 urine samples. In 1 urine sample with ST crystals in the sediment, supernatant crystals ST were detected in that sample, and IR analysis of calculus components detected ST in that patient's urinary calculus components. In 1 urine sample with CYS crystals in the sediment, supernatant crystals CYS were detected in that sample, and IR analysis of calculus components detected CYS in that patient's urinary calculus components.

In summary, when any of the 5 types of urolithiasis-related crystals are present (+) in the sediment, the supernatant will show the same type of crystals 100% of the time, and the probability that the correponding patient's calculus components contain the same crystal component is extremely high (out of a total of 24 patients, only 2 are exceptions).

**Table 6. Urine samples without Sediment Crystals but with Supernatant Crystals and Calculus Analysis Results**

| | | Respective Numbers in Urine samples and in Patient's Calculus Components | | | | |
|---|---|---|---|---|---|---|
| | | CaOx | UA | CaP | ST | CYS |
| Sediment Crystals (-) | | 40 | 1 | 8 | 2 | 1 |
| Supernatant Crystals | (+) | 40 | 1 | 8 | 2 | 1 |
| | (- ) | 0 | 0 | 0 | 0 | 0 |
| IR Analysis of Patient's Calculus | (+) | 37 | 1 | 7 | 2 | 1 |
| Components | (-) | 3 | 0 | 1 | 0 | 0 |

Table 6 shows that in 40 urine samples without CaOx crystals in the sediment but with CaOx detected in supernatant crystals, and IR analysis of calculus components CaOx were detected in 37 patient's urinary calculus components, with only 3 exceptions (CaOx components were not detected in 3 patient's urinary calculus components). In 1 urine sample without UA crystals in the sediment but with UA detected in supernatant crystals, and IR analysis of calculus components detected UA in that patient's urinary calculus components. In 8 urine samples without CaP crystals in the sediment but with CaP detected in supernatant crystals, and IR analysis of calculus components detected CaP in 7 patient's urinary calculus components, with 1 exception. In 2 urine samples without ST crystals in the sediment but with ST detected in supernatant crystals, and IR analysis of calculus components detected ST in all 2 patient's urinary calculus components. In 1 urine sample without CYS crystals in the sediment but with CYS detected in supernatant crystals, and IR analysis of calculus components detected CYS in that patient's urinary calculus components.

In summary, when no urolithiasis-related crystals (-) are present in the sediment, but a certain type of urolithiasis-related crystal appears (+) in the supernatant, there is a higher probability of consistency with the patient's calculus component analysis. That is, if any of CaOx, UA, CaP, ST, CYS crystals are present (+) in the supernatant crystals, the likelihood of the same component contained in urinary calculus of the corresponding patient is 92.5%, 100.0%, 87.5%, 100.0%, 100.0%, respectively.

The comprehensive results of this embodiment indicate that, (1) when a certain type of urolithiasis-related crystal is already present (+) in the urinary sediment, it indicates stage 2 of calculus formation, that is, this type of solid crystal in the urine may further form calculi, and the urolithiasis risk for this type of component is classified as high risk; (2) if no urolithiasis-related crystals are present in the urinary sediment, but a certain type of urolithiasis-related crystal appears (+) in the supernatant upon processing, it indicates stage 1 of calculus formation, the urine has the potential to form this type of crystal, and the urolithiasis risk for this type of component is classified as medium risk; (3) when neither urinary sediment nor supernatant contains urolithiasis-related crystals, but considering that the concentration of urine produced by different parts of each kidney is not completely consistent, there is still a possibility of higher concentration urine in some kidney areas, and the possibility of crystal formation cannot be completely ruled out, therefore, the urolithiasis risk is classified as low risk. Urine dry chemistry, pH value, specific gravity, osmotic pressure, electrical conductivity, etc., are auxiliary indicators and factors that promote or inhibit the formation of a specific type of crystal and its subsequent induction of urolithogenesis.

### Embodiment 4

Embodiment 4 is essentially the same as Embodiment 1, with the main difference being that the supernatant crystallization processing in Embodiment 4 can be carried out at temperatures of 37-55°C, for example, at 55°C, as shown in the results in the tables below. The urine dry chemistry and urinary sediment detection are substantially the same as in Embodiment 1.

In this Embodiment, urine samples with sediment crystals in the urinary sediment, and urine samples without sediment crystals in the urinary sediment but with urolithiasis-related crystals in the supernatant, were selected respectively, and the crystals were compared and analyzed with patient's urinary calculus components of corresponding urine samples, respectively. The analysis results are as follows.

**Table 7. Supernatant Crystallization of Urine samples with Sediment Crystals and Calculus Analysis Results**

| | | Respective Numbers in Urine samples and in Patient's Calculus Components | | | | |
|---|---|---|---|---|---|---|
| | | CaOx | UA | CaP | | |
| Sediment Crystals (+) | | 12 | 2 | 1 | | |
| Supernatant Crystals | (+) | 9 | 1 | 1 | | |
| | (- ) | 3 | 1 | 0 | | |
| IR Analysis of Patient's Calculus Components | (+) | 11 | 1 | 1 | | |
| | (- ) | 1 | 1 | 0 | | |

Table 7 shows that in 12 urine samples with CaOx crystals in the sediment, supernatant crystals CaOx were detected in 9 urine samples, and IR analysis of calculus components detected CaOx in 11 patient's urinary calculus components, with 1 exception (CaOx components were not detected in 1 patient's urinary calculus components). In 2 urine samples with UA crystals in the sediment, supernatant crystals UA were detected in 1 sample, and IR analysis of calculus components detected UA in 1 patient's urinary calculus components. In 1 urine sample with CaP crystals in the sediment, supernatant crystals CaP were detected in that sample, and IR analysis of calculus components detected CaP in that patient's urinary calculus components.

In summary, when any of the 3 types of urolithiasis-related crystals are present (+) in the sediment, the supernatant will show the same type of crystals 73.3% of the time, and the probability that the corresponding patient's calculus components contain the same crystal component is very high (out of a total of 15 patients, only 2 are exceptions).

**Table 8. Urine samples without Sediment Crystals but with Supernatant Crystals and Calculus Analysis Results**

| | | Respective Numbers in Urine samples and in Patient's Calculus Components | | | | |
|---|---|---|---|---|---|---|
| | | CaOx | UA | CaP | | |
| Sediment Crystals (-) | | 12 | 1 | 1 | | |
| Supernatant Crystals | (+) | 12 | 1 | 1 | | |
| | (- ) | 0 | 0 | 0 | | |
| IR Analysis of Patient's Calculus Components | (+) | 11 | 1 | 0 | | |
| | (- ) | 1 | 0 | 1 | | |

Table 8 shows that in 12 urine samples without CaOx crystals in the sediment but with CaOx detected in supernatant crystals, and IR analysis of calculus components detected CaOx in 11 patient's urinary calculus components, with only 1 exception (CaOx components were not detected in 1 patient's urinary calculus components). In 1 urine sample without UA crystals in the sediment but with UA detected in supernatant crystals, and IR analysis of calculus components detected UA in that patient's urinary calculus components. In 1 urine sample without CaP crystals in the sediment but with CaP detected in supernatant crystals, and IR analysis of calculus components detected no CaP in that patient's urinary calculus components.

In summary, when no urolithiasis-related crystals (-) are present in the sediment, but a certain type of urolithiasis-related crystal (+) appears in the supernatant, there is a higher probability of consistency with the patient's calculus component analysis. That is, if any of CaOx, UA crystals are present (+) in the supernatant crystals, the likelihood the same component contained in urinary calculus of the corresponding patient is high.

The comprehensive results of this embodiment indicate that, (1) when a certain type of urolithiasis-related crystal is already present (+) in the urinary sediment, it indicates stage 2 of calculus formation, that is, this type of solid crystal in the urine may further form calculi, and the urolithiasis risk for this type of component is classified as high risk; (2) if no urolithiasis-related crystals are present in the urinary sediment, but a certain type of urolithiasis-related crystal appears (+) in the supernatant upon processing, it indicates stage 1 of calculus formation, the urine has the potential to form this type of crystal, and the urolithiasis risk for this type of component is classified as medium risk; (3) when neither urinary sediment nor supernatant contains urolithiasis-related crystals, but considering that the concentration of urine produced by different parts of each kidney is not completely consistent, there is still a possibility of higher concentration urine in some kidney areas, and the possibility of crystal formation cannot be completely ruled out, therefore, the urolithiasis risk is classified as low risk. Urine dry chemistry, pH value, specific gravity, osmotic pressure, electrical conductivity, etc., are auxiliary indicators and factors that promote or inhibit the formation of a specific type of crystal and its subsequent induction of urolithogenesis.

### Embodiment 5

Multiple patients with a history of urolithiasis treatment and calculus analysis reports were recruited. The morning urine of each patient was taken for urine dry chemistry analysis, urinary sediment crystallization analysis, and supernatant crystallization analysis, and compared with the patient's calculus analysis results to see if they contained the same type of crystals.

A urine testing system is used in this Embodiment, with the following modules and steps being executed as shown in FIG. 2.

Dry Chemistry-related Parameter Acquisition Module: Urine is subjected to dry chemistry analysis to obtain indicators such as the pH value, specific gravity, osmotic pressure, and electrical conductivity of the urine. Urine dry chemical indicators play an important role in urolithogenesis and urolithiasis recurrence risk control. For example, an acidic pH value tends to induce formation of calcium oxalate, uric acid, and cystine calculi, while an alkaline pH value tends to induce formation of calcium phosphate and magnesium ammonium phosphate calculi. A high specific gravity and a high osmotic pressure indicate a higher solute concentration in the urine, and a high electrical conductivity indicates a higher concentration of electrolytes in the urine, all of which are risk factors influencing the urolithogenesis and the urolithiasis recurrence .

Urine Processing Module: 5 ml of urine is drawn and subjected to centrifugation, sedimentation, filtration, or natural sedimentation, to separate the urine into sediment and supernatant.

Urinary Sediment Analysis Module: Sediment crystals in the urinary sediment are subjected to qualitative and/or quantitative analysis by image recognition or Raman spectroscopy. Qualitative analysis determines the presence and the type of sediment crystals, with the presence of crystals indicating a high risk, and analyzes the type of sediment crystals (common crystal types in urinary sediment are shown in FIGs. 3A-3F, where FIG. 3A shows CaOx crystals in the form of octahedral C2, equilateral rhombic C3, and elliptical C1; FIG. 3B shows CaOx crystals in the form of elliptical C1 and dumbbell shaped C4; FIG. 3C shows UA crystals in the form of irregular rhombic U3, square U1, and blocky U2; FIG. 3D shows CaP crystals in the form of rod shaped P1, bar shaped P2, and fagot shaped P3; FIG. 3E shows ST crystals in the form of roof shaped T1; FIG. 3F shows CYS crystals in the form of regular hexagonal Y2 and multilayered hexagonal Y1). Quantitative analysis involves counting and measuring the size of crystals that are positive (+) in qualitative analysis as well as other statistical treatment, and obtaining urine sediment-related parameters through qualitative and quantitative analysis.

Supernatant Analysis Module: Supernatant is processed and analyzed, by drawing a small amount of supernatant and then evaporation processing it at temperatures of 10-37 °C, such as 10 °C and 37°C, to form supernatant crystals through water loss. The formed supernatant crystals are subjected to qualitative analysis by Raman spectroscopy, infrared spectroscopy, or image recognition, that is, identifying the type, chemical component, and crystal structure of the supernatant crystals (common urolithiasis-related crystal types in supernatant are shown in FIGs. 4A-4H, where FIG. 4A shows CaOx crystals in the form of octahedral; FIG. 4B shows CaOx crystals in the form of equilateral rhombic; FIG. 4C shows CaOx crystals in the form of biconvex columnar; FIG. 4D shows CaOx crystals in the form of drum shaped; FIG. 4E shows UA crystals in the form of irregular rhombic, multilayered lamellar, and petal shaped; FIG. 4F shows CaP crystals in the form of rod shape, bar shape, fagot shape, feather shape, and chrysanthemum shape; FIG. 4G shows ST crystals in the form of box-lid shape, roof shape, envelope shape, and coffin-lid shape; FIG. 4H shows CYS crystals in the form of regular hexagonal shape and multilayered hexagonal shape), and obtaining supernatant-related parameters through quantitative analysis of crystal counting, crystal size and area proportion via image recognition.

Data Processing Module: Data Processing Module assesses the urolithiasis etiology, urinary calculus component, and urolithiasis risk based on supernatant-related parameters, urinary sediment-related parameters, and dry chemistry-related parameters.

According to the current research and theoretical consensus in the medical community on the urolithogenesis (such as the references of "International Urolithiasis Alliance: Urolithiasis Metabolism and Management Guidelines 2022"), the urolithogenesis, i.e., the formation of urinary calculi, is closely related to the sediment crystals and the saturation degree of calculus-related components in urine, that is, (1) the sediment crystals in urine sediment are crystals that already exist in urine, indicating that the components of these crystals are already oversaturated in urine (at least locally oversaturated while secreted by local parts of kidney to form urine), and are an important cause of urinary calculus formation, (2) the oversaturation of calculus-related components in supernatant is prone to induce precipitatation and formation of crystals, which is thus closely related to urolithogenesis.

In this Embodiment, urine samples with sediment crystals in the urinary sediment, and urine samples without sediment crystals in the urinary sediment but with urolithiasis-related crystals in the supernatant, were selected respectively, and these crystals were compared and analyzed with the patient's urinary calculus components of corresponding urine samples, respectively. The analysis results are listed as follows.

**Table 9. Supernatant Crystallization of Urine samples with Sediment Crystals and Calculus Analysis Results**

| | | Respective Numbers in Urine samples and in Patient's Calculus Components | | | | |
|---|---|---|---|---|---|---|
| | | CaOx | UA | CaP | ST | CYS |
| Sediment Crystals (+) | | 52 | 8 | 3 | 2 | 1 |
| Supernatant Crystals | (+) | 52 | 8 | 3 | 2 | 1 |
| | (-) | 0 | 0 | 0 | 0 | 0 |
| IR Analysis of Patient's Calculus Components | (+) | 51 | 8 | 3 | 2 | 1 |
| | (-) | 1 | 0 | 0 | 0 | 0 |

Table 9 shows that in 52 urine samples with CaOx crystals in the sediment, supernatant crystals CaOx were detected in all 52 samples, and IR analysis of calculus components of patients have detected CaOx in 51 patient's urinary calculus components, with only 1 exception (calculus component analysis did not detect out CaOx in only 1 patient's urinary calculus components). In 8 urine samples with UA crystals in the sediment, supernatant crystals UA are detected in all 8 samples, and IR analysis of calculus components detected UA in all 8 patient's urinary calculus components. In 3 urine samples with CaP crystals in the sediment, supernatant crystals CaP are detected in all the 3 samples, and IR analysis of calculus components detected CaP in 3 patient's urinary calculus components. In 2 urine samples with ST crystals in the sediment, supernatant crystals ST are detected in these 2 urine samples, and IR analysis of calculus components detected ST in 2 patient's urinary calculus components. In 1 urine sample with CYS crystals in the sediment, supernatant crystals CYS are detected in that 1 sample, and IR analysis of calculus components detected CYS in 1 patient's urinary calculus components.

In summary, when any of the 5 types of urolithiasis-related crystals are present (+) in the sediment, the supernatant will show the same type of crystals 100% of the time, and the probability that the patient's calculus components contain the same component (s) is extremely high (out of a total of 66 patients, only 1 is an exception).

**Table 10. Urine samples without Sediment Crystals but with Supernatant Crystals and Calculus Analysis Results**

| | | Respective Numbers in Urine samples and in Patient's Calculus Components | | | | |
|---|---|---|---|---|---|---|
| | | CaOx | UA | CaP | ST | CYS |
| Sediment Crystals (-) | | 126 | 3 | 26 | 5 | 2 |
| Supernatant Crystals | (+) | 126 | 3 | 26 | 5 | 2 |
| | (- ) | 0 | 0 | 0 | 0 | 0 |
| IR Analysis of Patient's Calculus Components | (+) | 118 | 2 | 21 | 4 | 2 |
| | (- ) | 8 | 1 | 5 | 1 | 0 |

Table 10 shows that in 126 urine samples without CaOx crystals in the sediment but with CaOx detected in supernatant crystals, and IR analysis of calculus components detected CaOx in 118 patient's urinary calculus components, with only 8 exceptions ( No CaOx being detected in 8 patient's urinary calculus components). In 3 urine samples without UA crystals in the urine sediment but with UA crystals detected in supernatant crystals, and IR analysis of calculus components detected UA in 2 patient's urinary calculus components, with 1 exception without UA detected. In 26 urine samples without CaP crystals in the sediment but with CaP detected in supernatant crystals, and IR analysis of calculus components detected CaP in 21 patient's urinary calculus components, with 5 exceptions. In 5 urine samples without ST crystals in the sediment but with ST detected in supernatant crystals, and IR analysis of calculus components detected ST in 4 patient's urinary calculus components, with 1 exception. In 2 urine samples without CYS crystals in the sediment but with CYS detected in supernatant crystals, and IR analysis of calculus components detected CYS in all 2 patient's urinary calculus components.

In summary, when no urolithiasis-related crystals (-) are present in the sediment , but a certain type of urolithiasis-related crystal appears (+) in the supernatant, there is a higher probability of consistency with the patient's calculus component analysis. That is, if any one of CaOx, UA, CaP, ST, and CYS crystals is present (+) in the supernatant crystals, the likelihood of the same component contained in urinary calculus of the corresponding patient is 93.7%, 66.7%, 80.8%, 80.0%, 100%, respectively.

The comprehensive results of this embodiment indicate that, (1) when a certain type of urolithiasis-related crystal is already present (+) in the urinary sediment, it indicates stage 2 of calculus formation, that is, this type of solid crystal in the urine may further form calculi, and the urolithiasis risk for this type of component is classified as high risk; (2) if no urolithiasis-related crystals are present in the urinary sediment, but a certain type of urolithiasis-related crystal appears (+) in the supernatant upon processing, it indicates stage 1 of calculus formation, the urine has the potential to form this type of crystal, and the urolithiasis risk for this type of component is classified as medium risk; (3) when neither urinary sediment nor supernatant contains urolithiasis-related crystals, but considering that the concentration of urine produced by different parts of each kidney is not completely consistent, there is still a possibility of higher concentration urine in some kidney areas, and the possibility of crystal formation cannot be completely ruled out, therefore, the urolithiasis risk is classified as low risk. Urine dry chemistry, pH value, specific gravity, osmotic pressure, electrical conductivity, etc., are auxiliary indicators and factors that promote or inhibit the formation of a specific type of crystal and its subsequent induction of urolithogenesis.

### Embodiment 6

Embodiment 6 is essentially the same as Embodiment 5, with the main difference being that the supernatant crystallization processing in Embodiment 6 is carried out at a temperature of 4-10°C, such as at 4°C, with the results as shown in the tables below. The urine dry chemistry and urinary sediment detection are substantially the same as in Embodiment 5.

In this Embodiment 6, urine samples with sediment crystals in the urinary sediment, and urine samples without sediment crystals in the urinary sediment but with urolithiasis-related crystals in the supernatant, were selected respectively, and these crystals were compared and analyzed with the patient's urinary calculus components of corresponding urine samples, respectively. The analysis results are listed as follows.

**Table 11. Supernatant Crystallization of Urine samples with Sediment Crystals and Calculus Analysis Results**

| | | Respective Numbers in Urine samples and in Patient's Calculus Components | | | | |
|---|---|---|---|---|---|---|
| | | CaOx | UA | CaP | | |
| Sediment Crystals (+) | | 25 | 3 | 2 | | |
| Supernatant Crystals | (+) | 25 | 3 | 2 | | |
| | (- ) | 0 | 0 | 0 | | |
| IR Analysis of Patient's Calculus Components | (+) | 25 | 3 | 1 | | |
| | (- ) | 0 | 0 | 1 | | |

Table 11 shows that in 25 urine samples with CaOx crystals in the sediment, supernatant crystals CaOx were detected in all 25 samples, and IR analysis of calculus components of patients have detected CaOx in all 25 patient's urinary calculus components, with no exceptions (0 urinary calculus component did not contain CaOx components in the analysis). In 3 urine samples with UA crystals in the sediment, supernatant crystals UA were detected in all 3 samples, and IR analysis of calculus components detected UA in all 3 patient's urinary calculus components. In 2 urine samples with CaP crystals in the sediment, supernatant crystals CaP were detected in the 2 urine samples, and IR analysis of calculus components detected CaP in 1 patient's urinary calculus components.

In summary, when any of the 3 types of urolithiasis-related crystals are present (+) in the sediment, the supernatant will show the same type of crystals 100% of the time, and the probability that the patient's calculus components contain the same crystal component is extremely high (out of a total of 30 patients, only 1 is an exception).

**Table 12. Urine samples without Sediment Crystals but with Supernatant Crystals and Calculus Analysis Results**

| | | Respective Numbers in Urine samples and in Patient's Calculus Components | | | | |
|---|---|---|---|---|---|---|
| | | CaOx | UA | CaP | | |
| Sediment Crystals (-) | | 20 | 4 | 2 | | |
| Supernatant Crystals | (+) | 20 | 4 | 2 | | |
| | (- ) | 0 | 0 | 0 | | |
| IR Analysis of Patient's Calculus Components | (+) | 18 | 3 | 1 | | |
| | (- ) | 2 | 1 | 1 | | |

Table 12 shows that in 20 urine samples without CaOx crystals in the sediment but with CaOx detected in supernatant crystals, and IR analysis of calculus components detected CaOx in 18 calculus components of patients, with only 2 exceptions (CaOx component were not detected in 2 calculus components of patients). In 4 urine samples without UA crystals in the sediment but with UA detected in supernatant crystals, and IR analysis of calculus components detected UA in 3 calculus components of patients. In 2 urine samples without CaP crystals in the sediment but with CaP detected in supernatant crystals, and IR analysis of calculus components detected CaP in 1 calculus component of patient, with 1 exception.

In summary, when urolithiasis-related crystals are not present (-) in the sediment, but a certain type of urolithiasis-related crystal appears (+) in the supernatant, there is a higher probability of consistency with the corresponding patient's calculus component analysis. That is, if any one of CaOx, UA, CaP crystals is present (+) in the supernatant crystals, the likelihood of the same component contained in urinary calculus of the corresponding patient is 90.0%, 75.0%, 50.0%, respectively.

The comprehensive results of this Embodiment indicate that, (1) when a certain type of urolithiasis-related crystal is already present (+) in the urinary sediment, it indicates stage 2 of calculus formation, that is, this type of solid crystal in the urine may further form calculi, and the urolithiasis risk for this type of component is classified as high risk; (2) if no urolithiasis-related crystals are present in the urinary sediment, but a certain type of urolithiasis-related crystal appears (+) in the supernatant after processing, it indicates stage 1 of calculus formation, the urine has the potential to form this type of crystal, and the urolithiasis risk for this component is classified as medium risk; (3) when neither urinary sediment nor supernatant contains urolithiasis-related crystals, but considering that the concentration of urine produced by different parts of each kidney is not completely consistent, there is still a possibility of higher concentration urine in some kidney areas, and the possibility of crystal formation cannot be completely ruled out, therefore, the urolithiasis risk is classified as low risk. Urine dry chemistry, pH value, specific gravity, osmotic pressure, electrical conductivity, etc., are auxiliary indicators and factors that promote or inhibit the formation of a specific type of crystal and its subsequent induction of urolithogenesis.

### Embodiment 7

Embodiment 7 is essentially the same as Embodiment 5, with the difference being that the supernatant crystallization processing in Embodiment 7 is carried out at temperatures of -10 - 4°C, such as at -10°C and 4°C. The urine dry chemistry and urinary sediment detection are identical to those in Embodiment 5.

In this Embodiment 7, urine samples with sediment crystals in the urinary sediment, and urine samples without sediment crystals in the urinary sediment but with urolithiasis-related crystals in the supernatant, were selected respectively, and these crystals were compared and analyzed with the patient's urinary calculus components of corresponding urine samples, respectively. The analysis results are listed as follows.

**Table 13. Supernatant Crystallization of Urine samples with Sediment Crystals and Calculus Analysis Results**

| | | Respective Numbers in Urine samples and in Patient's Calculus Components | | | | |
|---|---|---|---|---|---|---|
| | | CaOx | UA | CaP | ST | CYS |
| Sediment Crystals (+) | | 17 | 3 | 2 | 1 | 1 |
| Supernatant Crystals | (+) | 17 | 3 | 2 | 1 | 1 |
| | (-) | 0 | 0 | 0 | 0 | 0 |
| IR Analysis of Patient's Calculus Components | (+) | 15 | 3 | 2 | 1 | 1 |
| | (-) | 2 | 0 | 0 | 0 | 0 |

Table 13 shows that in 17 urine samples with CaOx crystals in the sediment, supernatant crystals CaOx were detected in all 17 samples, and IR analysis of calculus components detected CaOx in 15 patient's urinary calculus components, with 2 exceptions (in 2 patient's urinary calculus components, CaOx components were not detected). In 3 urine samples with UA crystals in the sediment, supernatant crystals UA were detected in all 3 urine samples, and IR analysis of calculus components detected UA in all 3 patient's urinary calculus components. In 2 urine samples with CaP crystals in the sediment, supernatant crystalsCaP were detected in these 2 urine samples, and IR analysis of calculus components detected CaP were detected in all the 2 urine samples. In 1 urine sample with ST crystals in the sediment, supernatant crystals ST were detected in that sample, and IR analysis of calculus components detected ST in that patient's urinary calculus components. In 1 urine sample with CYS crystals in the sediment, supernatant crystals CYS were detected in that sample, and IR analysis of calculus components detected CYS in that patient's urinary calculus components.

In summary, when any of the 5 types of urolithiasis-related crystals are present (+) in the sediment, the supernatant will show the same type of crystals 100% of the time, and the probability that the correponding patient's calculus components contain the same crystal component is extremely high (out of a total of 24 patients, only 2 are exceptions).

**Table 14. Urine samples without Sediment Crystals but with Supernatant Crystals and Calculus Analysis Results**

| | | Respective Numbers in Urine samples and in Patient's Calculus Components | | | | |
|---|---|---|---|---|---|---|
| | | CaOx | UA | CaP | ST | CYS |
| Sediment Crystals (-) | | 40 | 1 | 8 | 2 | 1 |
| Supernatant Crystals | (+) | 40 | 1 | 8 | 2 | 1 |
| | (-) | 0 | 0 | 0 | 0 | 0 |
| IR Analysis of Patient's Calculus Components | (+) | 37 | 1 | 7 | 2 | 1 |
| | (-) | 3 | 0 | 1 | 0 | 0 |

Table 14 shows that in 40 urine samples without CaOx crystals in the sediment but with CaOx detected in supernatant crystals, and IR analysis of calculus components CaOx were detected in 37 patient's urinary calculus components, with only 3 exceptions (CaOx components were not detected in 3 patient's urinary calculus components). In 1 urine sample without UA crystals in the sediment but with UA detected in supernatant crystals, and IR analysis of calculus components detected UA in that patient's urinary calculus components. In 8 urine samples without CaP crystals in the sediment but with CaP detected in supernatant crystals, and IR analysis of calculus components detected CaP in 7 patient's urinary calculus components, with 1 exception. In 2 urine samples without ST crystals in the sediment but with ST detected in supernatant crystals, and IR analysis of calculus components detected ST in all 2 patient's urinary calculus components. In 1 urine sample without CYS crystals in the sediment but with CYS detected in supernatant crystals, and IR analysis of calculus components detected CYS in that patient's urinary calculus components.

In summary, when no urolithiasis-related crystals (-) are present in the sediment, but a certain type of urolithiasis-related crystal appears (+) in the supernatant, there is a higher probability of consistency with the patient's calculus component analysis. That is, if any of CaOx, UA, CaP, ST, CYS crystals are present (+) in the supernatant crystals, the likelihood of the same component contained in urinary calculus of the corresponding patient is 92.5%, 100.0%, 87.5%, 100.0%, 100.0%, respectively.

The comprehensive results of this embodiment indicate that, (1) when a certain type of urolithiasis-related crystal is already present (+) in the urinary sediment, it indicates stage 2 of calculus formation, that is, this type of solid crystal in the urine may further form calculi, and the urolithiasis risk for this type of component is classified as high risk; (2) if no urolithiasis-related crystals are present in the urinary sediment, but a certain type of urolithiasis-related crystal appears (+) in the supernatant upon processing, it indicates stage 1 of calculus formation, the urine has the potential to form this type of crystal, and the urolithiasis risk for this type of component is classified as medium risk; (3) when neither urinary sediment nor supernatant contains urolithiasis-related crystals, but considering that the concentration of urine produced by different parts of each kidney is not completely consistent, there is still a possibility of higher concentration urine in some kidney areas, and the possibility of crystal formation cannot be completely ruled out, therefore, the urolithiasis risk is classified as low risk. Urine dry chemistry, pH value, specific gravity, osmotic pressure, electrical conductivity, etc., are auxiliary indicators and factors that promote or inhibit the formation of a specific type of crystal and its subsequent induction of urolithogenesis.

### Embodiment 8

Embodiment 8 is essentially the same as Embodiment 5, with the main difference being that the supernatant crystallization processing in Embodiment 8 is carried out at temperatures of 37-55°C, for example, at 55°C, as shown in the results in the tables below. The urine dry chemistry and urinary sediment detection are substantially the same as in Embodiment 5.

In this Embodiment, urine samples with sediment crystals in the urinary sediment, and urine samples without sediment crystals in the urinary sediment but with urolithiasis-related crystals in the supernatant, were selected respectively, and the crystals were compared and analyzed with patient's urinary calculus components of corresponding urine samples, respectively. The analysis results are as follows.

**Table 15. Supernatant Crystallization of Urine samples with Sediment Crystals and Calculus Analysis Results**

| | | Respective Numbers in Urine samples and in Patient's Calculus Components | | | | |
|---|---|---|---|---|---|---|
| | | CaOx | UA | CaP | | |
| Sediment Crystals (+) | | 12 | 2 | 1 | | |
| Supernatant Crystals | (+) | 9 | 1 | 1 | | |
| | (-) | 3 | 1 | 0 | | |
| IR Analysis of Patient's Calculus Components | (+) | 11 | 1 | 1 | | |
| | (-) | 1 | 1 | 0 | | |

Table 15 shows that in 12 urine samples with CaOx crystals in the sediment, supernatant crystals CaOx were detected in 9 urine samples, and IR analysis of calculus components detected CaOx in 11 patient's urinary calculus components, with 1 exception (CaOx components were not detected in 1 patient's urinary calculus components). In 2 urine samples with UA crystals in the sediment, supernatant crystals UA were detected in 1 sample, and IR analysis of calculus components detected UA in 1 patient's urinary calculus components. In 1 urine sample with CaP crystals in the sediment, supernatant crystals CaP were detected in that sample, and IR analysis of calculus components detected CaP in that patient's urinary calculus components.

In summary, when any of the 3 types of urolithiasis-related crystals are present (+) in the sediment, the supernatant will show the same type of crystals 73.3% of the time, and the probability that the corresponding patient's calculus components contain the same crystal component is very high (out of a total of 15 patients, only 2 are exceptions).

**Table 16. Urine samples without Sediment Crystals but with Supernatant Crystals and Calculus Analysis Results**

| | | Respective Numbers in Urine samples and in Patient's Calculus Components | | | | |
|---|---|---|---|---|---|---|
| | | CaOx | UA | CaP | | |
| Sediment Crystals (-) | | 12 | 1 | 1 | | |
| Supernatant Crystals | (+) | 12 | 1 | 1 | | |
| | (-) | 0 | 0 | 0 | | |
| IR Analysis of Patient's Calculus Components | (+) | 11 | 1 | 0 | | |
| | (-) | 1 | 0 | 1 | | |

Table 16 shows that in 12 urine samples without CaOx crystals in the sediment but with CaOx detected in supernatant crystals, and IR analysis of calculus components detected CaOx in 11 patient's urinary calculus components, with only 1 exception (CaOx components were not detected in 1 patient's urinary calculus components). In 1 urine sample without UA crystals in the sediment but with UA detected in supernatant crystals, and IR analysis of calculus components detected UA in that patient's urinary calculus components. In 1 urine sample without CaP crystals in the sediment but with CaP detected in supernatant crystals, and IR analysis of calculus components detected no CaP in that patient's urinary calculus components.

In summary, when no urolithiasis-related crystals (-) are present in the sediment, but a certain type of urolithiasis-related crystal (+) appears in the supernatant, there is a higher probability of consistency with the patient's calculus component analysis. That is, if any of CaOx, UA crystals are present (+) in the supernatant crystals, the likelihood the same component contained in urinary calculus of the corresponding patient is high.

The comprehensive results of this embodiment indicate that, (1) when a certain type of urolithiasis-related crystal is already present (+) in the urinary sediment, it indicates stage 2 of calculus formation, that is, this type of solid crystal in the urine may further form calculi, and the urolithiasis risk for this type of component is classified as high risk; (2) if no urolithiasis-related crystals are present in the urinary sediment, but a certain type of urolithiasis-related crystal appears (+) in the supernatant upon processing, it indicates stage 1 of calculus formation, the urine has the potential to form this type of crystal, and the urolithiasis risk for this type of component is classified as medium risk; (3) when neither urinary sediment nor supernatant contains urolithiasis-related crystals, but considering that the concentration of urine produced by different parts of each kidney is not completely consistent, there is still a possibility of higher concentration urine in some kidney areas, and the possibility of crystal formation cannot be completely ruled out, therefore, the urolithiasis risk is classified as low risk. Urine dry chemistry, pH value, specific gravity, osmotic pressure, electrical conductivity, etc., are auxiliary indicators and factors that promote or inhibit the formation of a specific type of crystal and its subsequent induction of urolithogenesis.

Those skilled in the art will appreciate that, without departing from the concept of the present application and being applicable, the technical features, parameters, numerical values, etc., of the above Embodiments can be combined in other feasible ways. For the sake of clarity, not all possible combinations of the technical features of the aforementioned Embodiments have been described herein. However, as long as there are no contradictions in the combinations of these technical features, parameters, numerical values, etc., they should be considered within the scope of the present disclosure. The foregoing description of several embodiments of the present application is provided for illustrative purposes. The description is not intended to be exhaustive, nor is it intended to limit the application to the precise parameters, values, steps, and/or forms disclosed herein. It is apparent that many modifications and variations are possible in light of the above teachings. The scope of the application and all equivalents are intended to be defined by the appended claims.

## Claims

1. A urine processing method, comprising the steps of:
S1: processing urine to obtain urinary sediment and supernatant; and
S2: performing crystallization processing on the supernatant and making analysis to obtain supernatant-related parameters.

2. The method of claim 1, wherein in the step S2, when supernatant crystals are formed in the supernatant upon crystallization processing, then making qualitative and/or quantitative analysis of the supernatant crystals to obtain the supernatant-related parameters, the supernatant-related parameters including types and/or quantities of the supernatant crystals.

3. The method of claim 2, wherein performing the crystallization processing on the supernatant at a temperature of -10°C - 55°C.

4. The method of claim 3, wherein performing evaporation-induced crystallization processing on the supernatant at a temperature of 4°C- 55°C.

5. The method of claim 3, wherein performing cold-induced crystallization processing on the supernatant or alternatively performing both evaporation-induced crystallization and cold-induced crystallization processing on the supernatant, at a temperature of -10°C- 4°C.

6. The method of any one of claims 1-5, wherein the method further comprises making dry chemistry analysis of the urine to obtain dry chemistry-related parameters.

7. The method of any one of claims 1-6, wherein the method further comprises identifying whether sediment crystals exist in the urinary sediment; and making analysis of the sediment crystals, if existed, to obtain urinary sediment-related parameters.

8. The method of claim 7, wherein making qualitative and/or quantitative analysis of the sediment crystals, to obtain the urinary sediment-related parameters, wherein the urinary sediment-related parameters including types and/or quantities of the sediment crystals.

9. The method of any one of claims 1-6, wherein the method is for processing single urine sample or combined multiple urine samples, wherein in the step S1, processing only single urine sample or alternatively combined multiple urine samples to obtain the urinary sediment and the supernatant.

10. The method of claim 7, wherein the method further comprises, comprehensively assessing urinary calculus components and urolithiasis risk, based on at least one of the supernatant-related parameters and the urinary sediment-related parameters.

11. The method of claim 7, wherein the method further comprises, comprehensively assessing the urinary calculus components and the urolithiasis risk, based on at least one of the supernatant-related parameters and the urinary sediment-related parameters, as well as the dry chemistry-related parameters.

12. A urine testing system, the urine testing system comprising:
a urine processing module configured to process urine to obtain urinary sediment and supernatant; and
a supernatant analysis module configured to perform crystallization processing on the supernatant and make analysis to obtain supernatant-related parameters.

13. The system of claim 12, wherein the system is configured to make qualitative and/or quantitative analysis of supernatant crystals to obtain the supernatant-related parameters when the supernatant crystals are formed in the supernatant upon crystallization processing, the supernatant-related parameters including types and/or quantities of the supernatant crystals.

14. The system of claim 13, wherein the the urine processing module is configured to perform crystallization processing on the supernatant at a temperature of -10°C - 55°C.

15. The system of claim 14, wherein the urine processing module is configured to perform evaporation-induced crystallization processing on the supernatant at a temperature of 4 °C- 55°C.

16. The system of claim 14, wherein the the urine processing module is configured to perform cold-induced crystallization processing on the supernatant at a temperature of -10 °C -4°C, or alternatively perform both evaporation-induced crystallization and cold-induced crystallization processing on the supernatant.

17. The system of any one of claims 12-16, wherein the system further comprises a dry chemistry-related parameter acquisition module configured to make dry chemistry analysis of the urine to obtain dry chemistry-related parameters.

18. The system of any one of claims 12-17, wherein the system further comprises a urinary sediment analysis module configured to identify whether sediment crystals exist in the urinary sediment, and to make analysis of the sediment crystals to obtain urinary sediment-related parameters.

19. The system of claim 18, wherein the system further comprises a data processing module configured to comprehensively assess the urinary calculus components and the urolithiasis risk, based on at least one of the supernatant-related parameters and the urinary sediment-related parameters.

20. The system of claim 18, wherein the system further comprises a data processing module configured to comprehensively assess the urinary calculus components and the urolithiasis risk, based on at least one of the supernatant-related parameters and the urinary sediment-related parameters, as well as the dry chemistry-related parameters.

21. The system of claim 18, wherein the system is configured to make qualitative and/or quantitative analysis of the sediment crystals to obtain the urinary sediment-related parameters, the urinary sediment-related parameters including types and/or quantities of the sediment crystals.

22. The system of claim 19 or 20, wherein the data processing module is configured to:
assess the urolithiasis risk as high risk when the sediment crystals are identified in the urinary sediment;
assess the urolithiasis risk as medium risk when the sediment crystals are not identified in the urinary sediment, and the supernatant crystals formed from the supernatant contain urolithiasis-related crystals; and
assess the urolithiasis risk as low risk when the sediment crystals are not identified in the urinary sediment, and the supernatant crystals formed from the supernatant contain none of the urolithiasis-related crystals.

23. The system of any one of claims 12-16, wherein the urine processing module is configured to process single urine sample or combined multiple urine samples to obtain the urinary sediment and the supernatant.

24. The system of any one of claims 12-16, wherein the supernatant analysis module is configured to identify, through Raman spectroscopy, infrared (IR) spectroscopy, or image recognition, whether the supernatant crystals contain at least one of the following urolithiasis-related crystals: CaOx (calcium oxalate), UA (uric acid), CaP ( calcium phosphate or brushite), ST (ammonium magnesium phosphate or triple phosphate), and CYS (cystine).

25. The system of claim 24, wherein the supernatant analysis module is configured to identify through image recognition, whether at least one of the following urolithiasis-related crystals: CaOx (calcium oxalate), UA (uric acid), CaP ( calcium phosphate or brushite), ST (ammonium magnesium phosphate or triple phosphate), and CYS (cystine), is existed, the image recognition comprises:
identifying a crystal as calcium oxalate when the image of said crystal is of at least one of shapes comprising octahedral shape, equilateral rhombic shape, elliptical shape, dumbbell shape, biconvex columnar shape, and drum shape;
identifying a crystal as uric acid when the image of said crystal is of at least one of shapes comprising irregular rhombic shape, square shape, blocky shape, multilayered lamellar shape, and petal shape;
identifying a crystal as calcium phosphate or brushite when the image of said crystal is of at least one of shapes comprising rod shape, bar shape, fagot shape, feather shape, and chrysanthemum shape;
identifying a crystal as ammonium magnesium phosphate or triple phosphate when the image of said crystal is of at least one of shapes comprising box-lid shape, roof shape, envelope shape, and coffin-lid shape; and
identifying a crystal as cystine when the image of said crystal is of at least one of shapes comprising regular hexagonal shape and multilayered hexagonal shape.
